(19) Europäisches Patentamt
European Patent Office
Office européen des brevets

(11) Veröffentlichungsnummer: **0 354 991 B1**

# (12) EUROPÄISCHE PATENTSCHRIFT

(45) Veröffentlichungstag der Patentschrift: **05.05.93**

(51) Int. Cl.[5]: **C07C 45/62**, C07C 49/233, C07C 49/235, C07C 49/813, C07C 49/567

(21) Anmeldenummer: **89112404.2**

(22) Anmeldetag: **07.07.89**

## (54) Verfahren zur Hydrierung von alfa, beta-ungesättigten Ketonen.

(30) Priorität: **20.07.88 DE 3824625**

(43) Veröffentlichungstag der Anmeldung:
**21.02.90 Patentblatt 90/08**

(45) Bekanntmachung des Hinweises auf die Patenterteilung:
**05.05.93 Patentblatt 93/18**

(84) Benannte Vertragsstaaten:
**BE CH DE FR GB IT LI NL**

(56) Entgegenhaltungen:
**FR-A- 2 362 133**
**US-A- 3 574 764**

(73) Patentinhaber: **BAYER AG**

**W-5090 Leverkusen 1 Bayerwerk(DE)**

(72) Erfinder: **Kiel, Wolfgang, Dr.**
**Carl-Leverkus-Strasse 37**
**W-5068 Odenthal(DE)**
Erfinder: **Ziemann, Heinz, Dr.**
**Am Wiesenberg 10**
**W-5653 Leichlingen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft ein Verfahren zur katalytischen Hydrierung der C–C–Doppelbindung von $\alpha,\beta$–ungesättigtenKetonen, die wenigstens einen Substituenten mit gebundenem Halogen haben, zu den entsprechenden gesättigten Ketonen. Diese Hydrierung wird an Ni–haltigen Katalysatoren und in Gegenwart einer organischen Schwefelverbindung durchgeführt.

Bei der selektiven Hydrierung von C=C–Bindungen in Gegenwart anderer funktioneller Gruppen ist beispielsweise die Labilität von Halogensubstituenten, insbesondere in basischem Milieu, zu beachten; selbst Fluor ist in Gegenwart von Nickel abspaltbar (Houben–Weyl, Methoden, 4. Aufl., Bd. 4/1c (1980), 155). Auch bei gleichzeitiger Anwesenheit von Carbonylgruppen, hier besonders bei $\alpha,\beta$–ungesättigten Ketonen, ist mit einer unerwünschten Hydrierung der Oxogruppe zu rechnen (Houben–Weyl, loc. cit., S. 162); diese unerwünschte Reaktion wird bei Ni–Katalysatoren stärker als bei Pd–Katalysatoren beobachtet (P. Rylander, Catalytic Hydrogenation in Organic Synthesis 1979, S. 51).

Auch bei der katalytischen Hydrierung aromatischer Nitroverbindungen zu den zugehörigen Arylaminen wird eine Abspaltung von gleichzeitig vorhandenem, aromatisch gebundenem Halogen beobachtet. Zur Unterdrückung einer solchen Halogenspaltung wurden die für solche Hydrierungen eingesetzten Edelmetall–Katalysatoren modifiziert. Hierzu kann so vorgegangen werden, daß ein separat hergestellter modifizierter Katalysator eingesetzt wird oder daß ein Modifizierungsmittel als solches zum Reaktionsansatz gegeben wird. So wurden in GB 1.064.959 Sulfide der Edelmetalle eingesetzt; DE–OS 21 05 780 beschreibt einen Pt/C–Kontakt mit $Na_2SO_3$–Dotierung; DE–OS 21 50 220 beschreibt Edelmetall/A–Kohle/Sulfoxid–Katalysatoren. DE–AS 25 49 900 beschreibt die Hydrierung von Chlor–Nitro–Aromaten mit Edelmetall–Katalysatoren und einem Zusatz von Thioethern als Modifizierungsmittel.

Der Gebrauch von Modifizierungsmitteln wie den geschilderten ist stets mit einer Verminderung der Katalysatoraktivität verbunden, die häufig zu längeren Hydrierzeiten führt oder die Anwendung schärferer Reaktionsbedingungen erfordert. Für die leicht verlaufende Hydrierung von Nitroverbindungen reicht nämlich die verminderte Katalysatoraktivität immer noch völlig aus, was für die Hydrierung von C=C–Doppelbindungen nicht zu erwarten war.

Bei komplizierten Hydrieraufgaben, wie beispielsweise der Hydrierung einer C=C–Doppelbindung unter Erhalt einer Oxogruppe und der Vermeidung einer hydrogenolytischen Abspaltung von Halogen, wie im vorliegenden Falle, war daher mit einem uneinheitlichen Verlauf der Hydrierung zu rechnen, insbesondere, da bei $\alpha,\beta$–ungesättigten Ketonen häufig entweder eine unerwünschte Hydrierung der Carbonylgruppe beobachtet wird oder zur Vermeidung dieser unerwünschten Reaktion eine unvollständige Hydrierung der Doppelbindung in Kauf genommen werden muß.

Für die Lösung dieser Aufgabe konnte das aus US 3 574 764 bekannte Verfahren der katalytischen Reduktion von ungesättigten Aldehyden zu gesättigten Aldehyden keinen Beitrag leisten, da hierin keine halogenhaltigen Substrate genannt werden, hingegen eine nur für Aldehyde typische Gefahr, nämlich die Abspaltung von Kohlenmonoxid, beachtet werden mußte. Dieses Verfahren arbeitet in der Gasphase bei Normaldruck mit $H_2$ und einem Ni–Katalysator, der als integrierten Bestandteil Schwefel oder anorganische Sulfide enthält.

Es wurde nun ein Verfahren zur katalytischen Hydrierung von $\alpha,\beta$–ungesättigten Ketonen der Formel

$$R^1-CH=CH-CO-R^2 \quad (I),$$

in der

$R^1$ und $R^2$ unabhängig voneinander für geradkettiges oder verzweigtes $C_1-C_{12}$–Alkyl oder $C_2-C_{12}$–Hydroxyalkyl, geradkettiges oder verzweigtes $C_2-C_{12}$–Alkenyl, $C_3-C_8$–Cycloalkyl, $C_3-C_8$–Cycloalkenyl, $C_7-C_{14}$–Aralkyl oder $C_6-C_{12}$–Aryl stehen, wobei mindestens einer der Reste $R^1$ und $R^2$ ein– bis dreifach durch Halogen substituiert ist,

zu den zugehörigen gesättigten Ketonen der Formel

$$R^{11}-CH_2-CH_2-CO-R^{12} \quad (II),$$

in der

$R^{11}$ und $R^{12}$ die Bedeutung von $R^1$ und $R^2$ annehmen mit der Ausnahme, daß Alkenyl und Cycloalkenyl zum zugehörigen Alkyl bzw. Cycloalkyl hydriert werden,

gefunden, das dadurch gekennzeichnet ist, daß ein Nihaltiger Katalysator eingesetzt wird und in flüssiger Phase bei einem $H_2$–Druck von 3–200 bar und in Gegenwart einer organischen Schwefelverbindung der Formel

$R^3-S(=O)_n-R^4$ (III)

gearbeitet wird, in der

$R^3$ und $R^4$ unabhängig voneinander geradkettiges oder verzweigtes $C_1-C_{12}$-Alkyl, Hydroxy-$C_2-C_{12}$-alkyl, Carboxy-$C_1-C_{12}$-alkyl oder Phenyl bedeuten und weiterhin

$R^3$ und $R^4$ gemeinsam für $-CH=CH-CH=CH-$, $-(CH_2)_4-$, $-(CH_2)_5-$, $-(CH_2)_2-S-(CH_2)_2-$ oder $-(CH_2)_2-O-(CH_2)_2-$ stehen können,

$R^4$ zusätzlich Wasserstoff oder $CO-C_1-C_{12}$-alkyl bedeuten kann und

n den Wert 0 oder 1 annimmt.

Geradkettiges oder verzweigtes $C_1-C_{12}$-Alkyl ist beispielsweise Methyl, Ethyl, Propyl, Isopropyl, Butyl, Isobutyl, tert.-Butyl, Amyl, Hexyl, Octyl, Decyl oder Dodecyl; bevorzugt ist $C_1-C_6$-Alkyl, besonders bevorzugt $C_1-C_4$-Alkyl. Hydkroxy-alkyl trägt an beliebiger Stelle, bevorzugt in $\omega$-Position, eine Hydroxygruppe und kann weiterhin durch Ether-Sauerstoff in der Kohlenstoffkette unterbrochen sein. Carboxy-alkyl trägt an beliebiger Stelle, bevorzugt in $\alpha$- oder $\omega$-Position, eine Carboxylgruppe. Es gelten die gleichen Vorzugsbereiche wie für Alkyl.

Geradkettiges oder verzweigtes $C_2-C_{12}$-Alkenyl ist beispielsweise Vinyl, Propenyl, Butenyl, Isobutenyl, Pentenyl, Hexenyl, Octenyl, Decenyl oder Dodecenyl; bevorzugt ist $C_2-C_6$-Alkenyl, besonders bevorzugt $C_2-C_4$-Alkenyl.

$C_3-C_8$-Cycloalkyl ist beispielsweise Cyclopropyl, Cyclobutyl, Cyclopentyl, Cyclohexyl, Cycloheptyl oder Cyclooctyl, das durch ein oder zwei Methyl- oder Ethylgruppen substituiert sein kann; bevorzugt sind substituiertes oder nicht substituiertes Cyclopropyl, Cyclopentyl oder Cyclohexyl.

$C_3-C_8$-Cycloalkenyl ist beispielsweise Cyclopropenyl, Cyclobutenyl, Cyclopentenyl, Cyclohexenyl, Cycloheptenyl oder Cyclooctenyl.

$C_7-C_{14}$-Aralkyl ist beispielsweise Benzyl, Phenylethyl, Naphthylmethyl, Naphthylethyl, Biphenylmethyl oder Biphenyl-ethyl, bevorzugt Benzyl oder Phenylethyl.

$C_6-C_{12}$-Aryl ist beispielsweise Phenyl, Naphthyl oder Biphenyl, bevorzugt Phenyl.

Mindestens einer der Reste im $\alpha,\beta$-ungesättigten Keton ist ein- bis dreifach durch Halogen, wie Fluor, Chlor oder Brom, bevorzugt Fluor oder Chlor, besonders bevorzugt Chlor, substituiert. Für den Fall der mehrfachen Substituenten kann es sich auch um eine Substitution durch verschiedene Halogenatome handeln.

Alle genannten aromatischen Reste können weiterhin eine oder zwei Methyl- oder Ethylgruppen, Methoxy- oder Ethoxygruppen sowie die Hydroxylgruppe tragen.

In bevorzugter Weise werden $\alpha,\beta$-ungesättigte Ketone der Formel

$R^5-CH=CH-CO-R^6$ (IV)

eingesetzt, in der

$R^5$ und $R^6$ unabhängig voneinander geradkettiges oder verzweigtes $C_1-C_6$-Alkyl, $C_2-C_6$-Alkenyl, Cyclopropyl, Cyclopentyl, Cyclohexyl, Benzyl, Phenylethyl oder Phenyl bedeuten, wobei, mindestens einer der Reste $R^5$ und $R^6$ für Phenyl steht und wobei weiterhin mindestens einer der Reste $R^5$ und $R^6$ einbis dreifach durch Halogen substituiert ist.

Weitere bevorzugte Ketone sind solche der Formel

$R^7-CH=CH-CO-R^8$ (IV),

in der

$R^7$ Phenyl bedeutet und

$R^8$ geradkettiges oder verzweigtes $C_1-C_6$-Alkyl, $C_2-C_6$-Alkenyl, Cyclopropyl, Benzyl oder Phenyl bedeutet,

wobei mindestens einer der Reste $R^7$ und $R^8$ ein- bis dreifach durch Halogen substituiert ist.

Weitere bevorzugte Ketone sind 5-Halogenphenyl-2,2-dimethyl-pent-4-en-3-one, besonders das 5-(4-Chlorphenyl)-2,2-dimethyl-pent-4-en-3-on, und 6-Halogenphenyl-3,3-dimethyl-hex-5-en-4-one.

Alle $\alpha,\beta$-ungesättigten Ketone sind jedenfalls durch mindestens einen Substituenten ausgezeichnet, der durch Halogen substituiert ist.

Die einzusetzenden $\alpha,\beta$-ungesättigten Ketone können über die bekannte Aldol-Kondensation hergestellt werden. Sie verfügen über 3 funktionelle Gruppen: C=C-Doppelbindung, Carbonylgruppe und

(mindestens ein) Halogen. Am Beispiel des 5 – (4 – Chlorphenyl) – 2,2 – dimethyl – pent – 4 – en – 3 – ons seien die Herstellung, die gewünschte Hydrierung und die unerwünschten Nebenreaktion dargestellt; im Falle eines alkoholischen Hydriermediums muß weiterhin die Alkoholaddition an die Doppelbindung be – fürchtet werden:

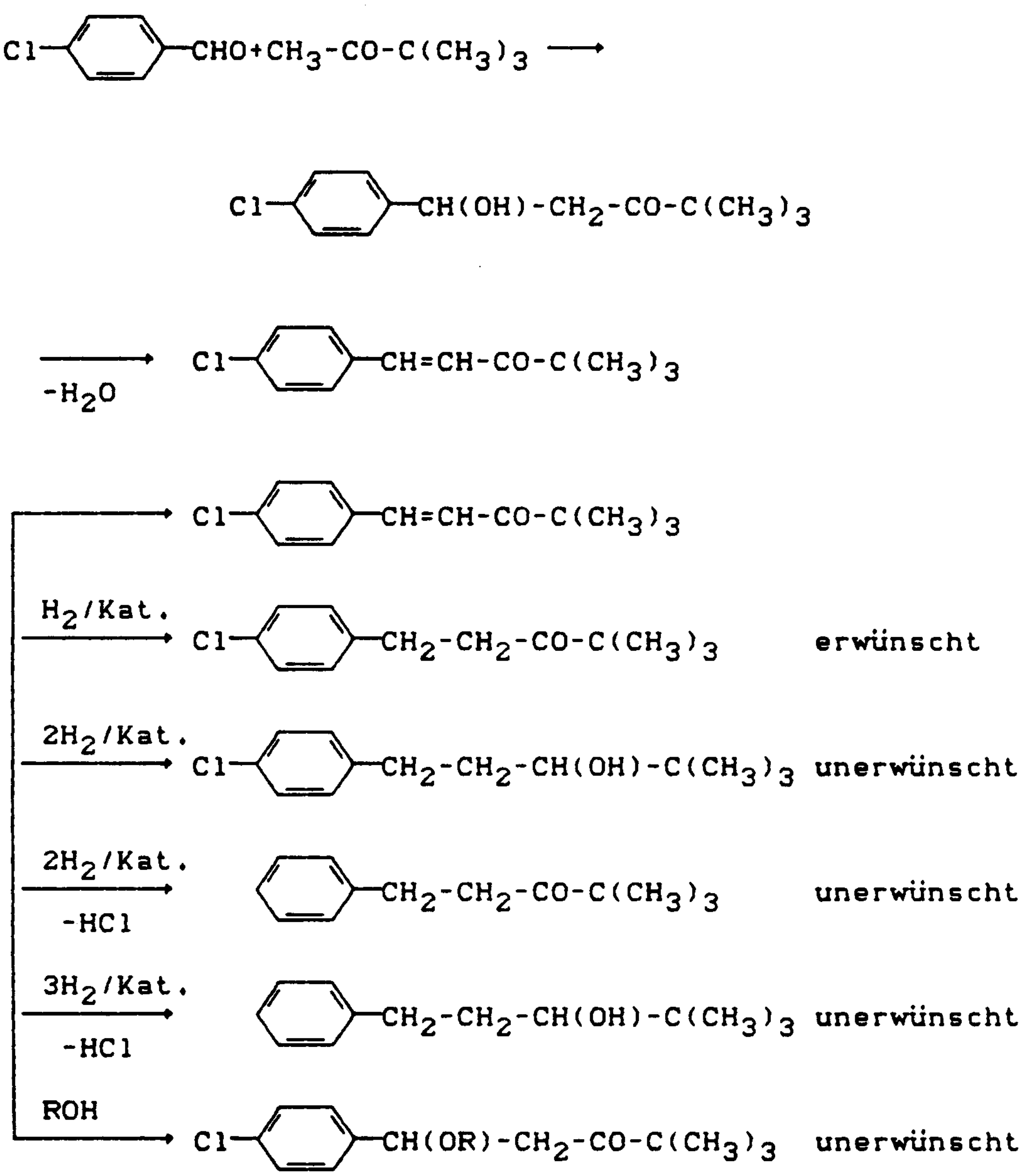


Hierbei ist es unerheblich, ob die unerwünschten Stoffe direkt aus dem $\alpha,\beta$ – ungesättigten Keton oder durch simultane Weiterreaktion des gewünschten gesättigten Ketons entstehen.

Das erfindungsgemäße Verfahren wird in Gegenwart einer oder mehrerer organischer Schwefelverbin – dungen der Formel (III) durchgeführt. Beispiele für solche Verbindungen sind Bis – (2 – hydroxyethyl) – sulfid, Bis – (2 – hydroxypropyl) – sulfid, Thiodiessigsäure und ihre Alkalimetallsalze, Thioanisol, Thiodipro – pionsäure, ihre Salze und ihre Dimethylester, Diphenylsulfid, Dithian, Thioxan, Thiophen, Benzthiazol, Dimethylsulfoxid, Methylethylsulfoxid, Diethylsulfoxid. Die organische Schwefelverbindung wird in einer Menge von 0,002 – 0,1 Gew. – Teile, bevorzugt 0,01 – 0,075 Gew. – Teile, je Gew. – Teil Katalysator einge – setzt.

In bevorzugter Weise wird eine organische Schwefelverbindung der Formel

$$R^{13} - S(=O)_n - R^{14}$$

eingesetzt, in der

$R^{13}$ und $R^{14}$ unabhängig voneinander Alkyl, Hydroxy-$C_2$-$C_{12}$-alkyl oder Carboxy-$C_1$-$C_{12}$-alkyl bedeuten und wobei

$R^{14}$ zusätzlich CO-$C_1$-$C_6$-alkyl bedeuten kann und

n den Wert 0 oder 1 annimmt.

In besonders bevorzugter Weise wird Bis-(2-hydroxyethyl)-sulfid eingesetzt.

Der Zusatz der organischen Schwefelverbindung erfolgt gemeinsam mit dem Katalysator, vor der Zugabe oder nach der Zugabe des Katalysators. Wird der Katalysator mehrfach wiederverwendet, braucht die organische Schwefelverbindung im allgemeinen nur beim ersten Einsatz dem Katalysator bzw. dem Reaktionsansatz zugesetzt zu werden. Der Katalysator behält dann auch nach vielfach wiederholter Verwendung oder bei einer kontinuierlichen Verfahrensweise über lange Zeit seine hohe spezifische Aktivität bei gleichbleibend hoher Ausbeute bei. Eine Nachdosierung der organischen Schwefelverbindung ist möglich, aber in der Regel nur erforderlich, wenn frischer Katalysator für etwa verbrauchten oder ausgeschleusten Katalysator zudosiert wird.

Als Hydrierkatalysatoren werden erfindungsgemäß Ni-haltige, wie Ni auf Trägern, Ni in Form von elementarem Nickelschwamm, Ni-oxid, Raney-Nickel und andere eingesetzt. Träger sind beispielsweise $SiO_2$, $Al_2O_3$, Bims, Kohle und andere dem Fachmann bekannte Träger. In bevorzugter Weise werden jedoch Raney-Katalysatoren, wie Raney-Nickel, Raney-Nickel-Eisen, Raney-Nickel-Kobalt oder Raney-Nickel-Eisen-Kobalt in wasserfreier oder auch wasserfeuchter oder Lösungsmittel-feuchter Form eingesetzt.

Als Reaktionsmedium können Alkohole, wie Methanol, Ethanol, Isopropanol, Butanol, aliphatische oder aromatische Kohlenwasserstoffe, wie Toluol, Xylol, Cyclohexan, Isooctan und ähnliche, Ether, wie Tetrahydrofuran, Dioxan oder Methyl-tert.-butylether, Ester, wie Ethylacetat und schließlich das Reaktionsprodukt selbst, sofern es bei der Reaktionstemperatur flüssig ist, eingesetzt werden. Ein Gehalt an Wasser (z. B. bis zu 20 Gew.-% des gesamten Reaktionsmediums) stört nicht, besonders, wenn das Reaktionsmedium wasserlöslich ist.

Die Hydrierung wird bei 30-250°C, bevorzugt bei 50-140°C, und einem $H_2$-Druck von 3-200 bar, bevorzugt 10-150 bar, durchgeführt. In einer bevorzugten Variante wird das erfindungsgemäße Verfahren durch den Zusatz eines basischen Stoffes bei einem pH-Wert von 8-14 durchgeführt. Basische Stoffe hierfür sind beispielsweise Natriumhydroxid, Kaliumhydroxid, Calciumhydroxid (etwa in der Form von gelöschtem Kalk), Calciumoxid, Kaliumcarbonat, Natriumcarbonat, Natriumacetat oder aliphatische oder heterocyclische Amine. Die basischen Stoffe können, soweit sie wasserlöslich, sind, als wäßrige Lösung oder als Substanz eingesetzt werden. In bevorzugter Weise wird wäßrige Natronlauge eingesetzt.

In überraschender Weise fördert ein solcher Zusatz von basischen Stoffen keineswegs die Halogenabspaltung. Des weiteren wird die bei Verwendung alkoholischer Reaktionsmedien beobachtete Nebenreaktion der Alkohol-Addition an C-C-Doppelbindung unterdrückt.

Der basisch reagierende Stoff wird in einer Menge von 0,001-0,025 Gew.-Teilen, vorzugsweise 0,002-0,01 Gew.-Teilen, je Gew.-Teil des $\alpha,\beta$-ungesättigten Ketons zugesetzt.

Im allgemeinen wird das Verfahren so durchgeführt, daß man den Ausgangsstoff, das Reaktionsmedium, den Katalysator, die organische Schwefelverbindung und gegebenenfalls die Base in einem Hydrierautoklaven vorlegt und nach Verschließen des Reaktors die Luft mit Stickstoff und anschließend den Stickstoff mit Wasserstoff verdrängt. Nach beendeter Reaktion wird das Reaktionsgefäß zunächst entspannt und entleert; der Katalysator wird abfiltriert und kann ohne neuen Zusatz von organischer Schwefelverbindung wiederverwendet werden.

Das Verfahren kann sowohl diskontinuierlich als auch kontinuierlich durchgeführt werden.

Das Verfahren kann weiterhin sowohl auf die reinen, ungesättigten $\alpha,\beta$-ungesättigten Ketone als auch auf ein rohes Reaktionsgemisch, beispielsweise aus der Aldol-Kondensation angewandt werden, in der solche $\alpha,\beta$-ungesättigten Ketone aus einem Aldehyd und einem Methylketon erhalten werden können.

Es ist überraschend, daß durch die organische Schwefelverbindung als Modifizierungsmittel, für die regelmäßig eine Desaktivierung beobachtet wird, die beschriebene Hydrierung selektiver, reproduzierbarer und vor allem vollständiger abläuft als ohne diese Zugabe; insbesondere ist es überraschend, daß die Hydrierung auch schneller abläuft als ohne Zugabe, d.h., die Hydrieraktivität erhöht wird, während sonst durch Schwefelverbindungen eine Desaktivierung erfolgt. Die Katalysatoren haben eine hohe Standzeit.

## Beispiele

### Allgemeine Arbeitsweise

In einem Autoklaven wurden die chlorierte α,β-ungesättigteKetoverbindung, das Lösungsmittel, der Katalysator sowie die organische Schwefelverbindung (mit Ausnahme der Vergleichsbeispiele) und gegebenenfalls eine Base vorgelegt.

Der Autoklav wurde zuerst mit Stickstoff und dann mit Wasserstoff gespült.

Anschließend wurde auf die angegebene Temperatur erhitzt und bei dem angegebenen Wasserstoffdruck hydriert, bis keine Wasserstoffaufnahme mehr erfolgte.

Nach Beendigung der Reaktion wurde der Katalysator abfiltriert; er konnte dann direkt für weitere Hydrierungen bereitgestellt werden. Aus dem Filtrat wurden nach üblichen Methoden (z.B. durch Destillation) die chlorierten gesättigten Ketone isoliert.

### Beispiele 1 bis 9 (Beispiele 1, 4, 6 und 8 zum Vergleich)

Hydrierung von 139 g 5-(4-Chlorphenyl)-2,2-dimethylpent-4-en-3-on in 245 g Methanol bzw. Toluol bei 100°C an 5,6 g Raney-Nickel mit und ohne Zusatz von Bis-(2-hydroxyethyl)-sulfid zu 5-(4-Chlorphenyl)-2,2-dimethylpentan-3-on (Endprodukt).

Der Katalysator enthielt entweder ca. 50 % Wasser oder 50 % Toluol.

Tabelle 1: Beispiele 1 - 9

| Beispiel Nr. | Lösungs-mittel | Zusatz | $H_2$ (bar) | Reaktions-zeit | dechlorierte Verbindung | Ausgangs-Verbindung | Endprodukt |
|---|---|---|---|---|---|---|---|
| 1 | Methanol | - | 150 | 10 Min. | 1,8 % | <0,1 % | 97,3 % |
| 2 | Methanol | 0,2 g | 150 | 3 Min. | 0,4 % | 0,1 5 | 99,3 % |
| 3 | Methanol | 0,7 g | 150 | 3 Min. | 0,1 % | <0,1 % | 99,1 % |
| 4 | Toluol | - | 150 | 20 Min. | 1,1 % | 6,0 % | 89,3 % |
| 5 | Toluol | 0,7 g | 150 | 3 Min. | 0,1 % | 0,1 % | 98,4 % |
| 6 | Methanol | - | 10 | 55 Min. | 1,6 % | 36,8 % | 60,7 % |
| 7 | Methanol | 0,7 g | 10 | 159 Min. | 0,3 % | 0,1 % | 99,0 % |
| 8 | Toluol | - | 10 | 15 Min. | 1,2 % | 73,5 % | 24,6 % |
| 9 | Toluol | 0,7 g | 10 | 41 Min. | 0,2 % | 0,1 % | 97,7 % |

Beispiele 10 und 11

Hydrierung von 139 g 5-(4-Chlorphenyl)-2,2-dimethylpent-4-en-3-on in 245 g Methanol bei 100 ° C und 150 bar $H_2$ an 5,6 g Raney-Nickel (wasserfeucht) mit Zusatz von 0,7 g Bis-(2-hydroxyethyl)-sulfid unter Zugabe von 45 %iger wäßriger Natronlauge. Vor Befüllung des Autoklaven wurde dieser Ansatz in einem Anmaischbehälter 30 Min. bei 80 ° C gerührt.

Tabelle 2

| Beispiele 10 und 11 | | | | | |
|---|---|---|---|---|---|
| Beisp. Nr. | NaOH (45 %ig) | dechlor Verb. | Methoxyaddukt an die C-C-Doppelbindung | Ausgangsverb. | Endprod. |
| 10 | - | 0,7 % | 6,0 % | 0,9 % | 92,0 % |
| 11 | 1,23 g | 0,4 % | - | 0,05 % | 99,0 % |

Beispiele 12 bis 19

Mehrfach wiederholte Hydrierung von 139 g 5-(4-Chlorphenyl)-2,2-dimethyl-pent-4-en-3-on in 245 g Methanol bei 100 ° C und 150 bar $H_2$ an jeweils zurückgewonnenem Raney-Nickel bei einmaligem Zusatz von 0,7 g Bis-(2-hydroxyethyl)-sulfid zum 1. Ansatz. Bei jedem Ansatz wurden 1,23 g 45 %ige NaOH-Lösung zugesetzt.

Tabelle 3: Beispiele 12 bis 19

| Beisp. | Katalysator, rückgewonnen | Reaktions-zeit | dechlorierte Verbindung | Methoxy-verb. | Ausgangs-verb. | Endprodukt |
|---|---|---|---|---|---|---|
| 12 | aus Beisp. 11 | 0,1 h | 0,3 % | - | <0,1 % | 99,3 % |
| 13 | aus Beisp. 12 | 0,1 h | 0,1 % | - | <0,1 % | 99,5 % |
| 14 | aus Beisp. 13 | 0,1 h | 0,1 % | - | <0,1 % | 99,5 % |
| 15 | aus Beisp. 14 | 0,1 h | 0,1 % | - | <0,1 % | 99,2 % |
| 16 | aus Beisp. 15 | 0,15 h | 0,2 % | - | <0,1 % | 99,1 % |
| 17 | aus Beisp. 16 | 0,18 h | 0,1 % | - | <0,1 % | 99,2 % |
| 18 | aus Beisp. 17 | 0,18 h | 0,1 % | - | <0,1 % | 98,6 % |
| 19 | aus Beisp. 18 | 0,18 h | 0,1 % | - | 0,4 % | 98,9 % |

Beispiele 20 - 26

In Anlehnung an die Arbeitsweise von Beispiel 1 wurden je 120 g 5-(4-Chlorphenyl)-2,2-dimethylpent-4-en-3-on in 180 g Methanol bei 110 °C und 150 bar $H_2$-Druck in Gegenwart der

angegebenen Zusätze hydriert. Die Hydrierzeit wurde einheitlich auf 6 min. festgelegt. Die Hydrierungen begannen im allgemeinen bei Raumtemperatur, erkennbar am $H_2$ – Verbrauch; in Beispiel 26 begann die Hydrierung bei 80 ° C, im Beispiel 24 bei 55 ° C. In der folgenden Tabelle sind die Ergebnisse zusammengestellt. Die Zusammensetzung der Reaktionsprodukte wurde gaschromatographisch bestimmt.

**Tabelle 4:** Beispiele 20 bis 26

| Beisp. | Zusatz Menge (g)/Art | Endprod. (%) | Ausgangs-verb. (%) | dechlorierte Verb. (%) | Methoxy-Verb. (%) | weitere Nebenprod. (%) |
|---|---|---|---|---|---|---|
| 20 | 0,2 $CH_3$-CO-SH | 98,9 | 0,1 | 0,6 | 0,3 | 0,1 |
| 21 | 0,2 HO-$C_2H_4$-SH | 99,3 | 0,1 | 0,4 | <0,1 | <0,1 |
| 22 | 0,2 HO-$C_2H_4$-S-$C_2H_5$ | 99,3 | 0,1 | 0,5 | 0,1 | - |
| 23 | 0,2 Thiophen | 98,9 | 0,1 | 0,6 | 0,3 | <0,1 |
| 24 | 0,2 S($CH_2$-COOH)$_2$ | 99,3 | 0,1 | 0,4 | 0,2 | - |
| 25 | 0,2 OS(CH)$_3$)$_2$ | 99,7 | <0,1 | 0,2 | <0,1 | <0,1 |
| 26 | 0,2 $CH_3COSC_6H_5$ | 99,8 | <0,1 | 0,1 | <0,1 | <0,1 |

Beispiel 27

Gemäß der im Beispiel 1 beschriebenen Arbeitsweise wurden 63 g des α,β-ungesättigten Ketons der Formel

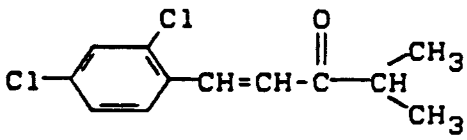

in 300 g Tetrahydrofuran an 10 g Raney-Nickel-Eisen mit Zusatz von 0,45 g Bis-(2-hydroxyethyl)-sulfid bei 50 °C und 80 bar Wasserstoffdruck innerhalb von 15 Minuten hydriert.

Das Reaktionsprodukt enthielt nach gaschromatographischer Analyse 97 % des gesättigten Ketons der Formel

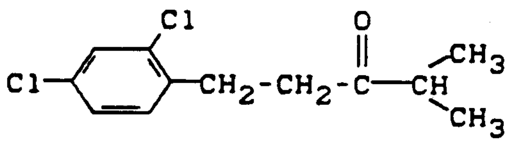

und nur 0,4 % dechlorierte Verbindungen.

Beispiel 28

Gemäß der in Beispiel 1 beschriebenen Arbeitsweise wurden 500 g des α,β-ungesättigten Ketons der Formel

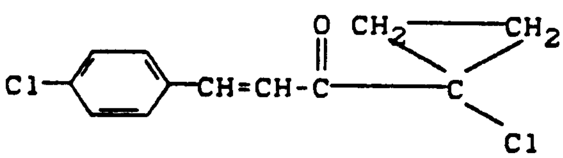

in 2000 g Methanol an 25 g Raney-Nickel mit Zusatz von 1,25 g Bis-(2-hydroxyethyl)-sulfid bei 70 °C und 80 bar Wasserstoffdruck innerhalb von 25 Minuten hydriert.

Das Reaktionsprodukt enthielt nach gaschromatographischer Analyse 98,5 % des gesättigten Ketons der Formel

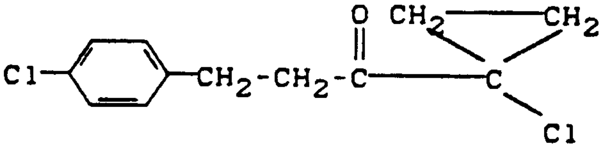

und nur 0,5 % dechlorierte Verbindungen.

Vergleichsbeispiele 29-32

Gemäß der im Beispiel 1 beschreibenen Arbeitsweise wurden jeweils 120 g 5-(4-Chlorphenyl)-2,2-dimethylpent-4-en-3-on in 210 g Methanol an 3 g 5 %iger Palladiumkohle in Gegenwart der in der Tabelle angegebenen Mengen Bis-(2-hydroxyethyl)-sulfid bei 50 °C und 100 bar $H_2$-Druck hydriert.

Die gaschromatographisch ermittelte Zusammensetzung der Reaktionsprodukte und die jeweiligen Hydrierzeiten sind in der folgenden Tabelle zusammengefaßt.

Die Ergebnisse der Beispiele 29 – 32 zeigen, daß mit einem Palladium – Katalysator unter Zusatz einer organischen Schwefelverbindung keine selektive Hydrierung der C,C – Doppelbindung in Gegenwart von Halogensubstituenten und der zum Carbinol hydrierbaren Ketongruppe möglich ist. Wie ersichtlich, gelingt es zwar, mit relativ hohen Zusätzen bei erheblich verlängerter Hydrierzeit die Dehalogenierung weitgehend zu unterbinden, aber die Hydrierung der Carbonylgruppe war nicht auszuschließen.

**Tabelle 5: Vergleichsbeispiele 29-32**

| Beispiel | Thioether [g] | Reaktionszeit [min] | Zusammensetzung des Reaktionsproduktes [%]* Endprodukt | Dechlorierte Verbindungen | Carbinol aus Ketogruppe |
|---|---|---|---|---|---|
| 29 | 0,015 | 10 | 38 | 19 | 40 |
| 30 | 0,03 | 15 | 49 | 8 | 41 |
| 31 | 0,15 | 80 | 43,5 | 4,5 | 50 |
| 32 | 0,30 | 105 | 74 | 0,7 | 24 |

* Ausgangsverbindung wurde nicht gefunden; der Rest zu 100 % sind nicht identifizierte Nebenprodukte

## Patentansprüche

**1.** Verfahren zur katalytischen Hydrierung von $\alpha,\beta$-ungesättigten Ketonen der Formel

$R^1-CH=CH-CO-R^2$,

in der

$R^1$ und $R^2$ unabhängig voneinander für geradkettiges oder verzweigtes $C_1-C_{12}$-Alkyl oder $C_2-C_{12}$-Hydroxyalkyl, geradkettiges oder verzweigtes $C_2-C_{12}$-Alkenyl, $C_3-C_8$-Cycloalkyl, $C_3-C_8$-Cycloalkenyl, $C_7-C_{14}$-Aralkyl oder $C_6-C_{12}$-Aryl stehen, wobei mindestens einer der Reste $R^1$ und $R^2$ ein- bis dreifach durch Halogen substituiert ist,

zu den zugehörigen gesättigten Ketonen der Formel

$R^{11}-CH_2-CH_2-CO-R^{12}$,

in der

$R^{11}$ und $R^{12}$ die Bedeutung von $R^1$ bzw. $R^2$ annehmen mit der Ausnahme, daß Alkenyl und Cycloalkenyl zum zugehörigen Alkyl bzw. Cycloalkyl hydriert werden,

dadurch gekennzeichnet, daß ein Ni-haltiger Katalysator eingesetzt wird und in flüssiger Phase bei einem $H_2$-Druck von 3-200 bar und in Gegenwart einer organischen Schwefelverbindung der Formel

$R^3 - S(=O)_n - R^4$

gearbeitet wird, in der

$R^3$ und $R^4$ unabhängig voneinander geradkettiges oder verzweigtes $C_1-C_{12}$-Alkyl, Hydroxy-$C_2-C_{12}$-alkyl, oder Carboxy-$C_1-C_{12}$-alkyl oder Phenyl bedeuten und weiterhin

$R^3$ und $R^4$ gemeinsam für $-CH=CH-CH=CH-$, $-(CH_2)_4-$, $-(CH_2)_5-$, $-(CH_2)_2-S-(CH_2)_2-$ oder $-(CH_2)_2-O-(CH_2)_2-$ stehen können,

$R^4$ zusätzlich Wasserstoff oder $CO-C_1-C_{12}$-alkyl bedeuten kann und

n den Wert 0 oder 1 annimmt.

2. Verfahren nach Anspruch 1, dadurch gekennzeichnet, daß organische Schwefelverbindungen der Formel

$R^{13} - S(=O)_n - R^{14}$

eingesetzt werden, in der

$R^{14}$ zusätzlich $CO-C_1-C_6$-alkyl bedeuten kann und

n den Wert 0 oder 1 annimmt.

3. Verfahren nach Anspruch 2, dadurch gekennzeichnet, daß Bis-(2-hydroxyethyl)-sulfid eingesetzt wird.

4. Verfahren nach Anspruch 1, dadurch gekennzeichnet, daß als Katalysator Raney-Nickel, Raney-Nickel-Eisen, Raney-Nickel-Kobalt oder Raney-Nickel-Eisen-Kobalt eingesetzt wird.

5. Verfahren nach Anspruch 1, dadurch gekennzeichnet, daß bei einem pH-Wert von 8-14 gearbeitet wird.

6. Verfahren nach Anspruch 1, dadurch gekennzeichnet, daß $\alpha,\beta$-ungesättigte Ketone der Formel

$R^5-CH=CH-CO-R^6$

eingesetzt werden, in der

$R^5$ und $R^6$ unabhängig voneinander geradkettiges oder verzweigtes $C_1-C_6$-Alkyl, $C_2-C_6$-Alkenyl, Cyclopropyl, Cyclopentyl, Cyclohexyl, Benzyl, Phenylethyl oder Phenyl bedeuten, wobei mindestens einer der Reste $R^5$ und $R^6$ für Phenyl steht und wobei weiterhin mindestens einer der Reste $R^5$ und $R^6$ ein- bis dreifach durch Halogen substituiert ist.

**7.** Verfahren nach Anspruch 6, dadurch gekennzeichnet, daß $\alpha,\beta$ − ungesättigte Ketone der Formel

$R^7 - CH=CH - CO - R^8$

eingesetzt werden, in der

$R^7$ Phenyl bedeutet und

$R^8$ geradkettiges oder verzweigtes $C_1-C_6$ − Alkyl, $C_2-C_6$ − Alkenyl, Cyclopropyl, Benzyl oder Phenyl bedeutet,

wobei mindestens einer der Reste $R^7$ und $R^8$ ein − bis dreifach durch Halogen substituiert ist.

**8.** Verfahren nach Anspruch 6, dadurch gekennzeichnet, daß 5 − Halogenphenyl − 2,2 − dimethyl − pent − 4 − en − 3 − one oder 6 − Halogenphenyl − 3,3 − dimethyl − hex − 5 − en − 4 − one eingesetzt werden.

**Claims**

**1.** Process for the catalytic hydrogenation of $\alpha,\beta$ − unsaturated ketones of the formula

$R^1-CH=CH-CO-R^2$,

in which

$R^1$ and $R^2$ independently of one another represent straight − chain or branched $C_1-C_{12}$ − alkyl or $C_2-C_{12}$ − hydroxyalkyl, straight − chain or branched $C_2-C_{12}$ − alkenyl, $C_3-C_8$ − cycloalkyl, $C_3-C_8$ − cycloalkenyl, $C_7-C_{14}$ − aralkyl or $C_6-C_{12}$ − aryl, where at least one of the radicals $R^1$ and $R^2$ is monosubstituted to trisubstituted by halogen,

to give the respective saturated ketones of the formula

$R^{11}-CH_2-CH_2-CO-R^{12}$,

in which

$R^{11}$ and $R^{12}$ assume the meaning of $R^1$ and $R^2$ with the exception that alkenyl and cycloalkenyl are hydrogenated to the respective alkyl or cycloalkyl,

characterized in that a Ni − containing catalyst is employed and that the reaction is carried out in the liquid phase at an $H_2$ pressure of 3 − 200 bar and in the presence of an organic sulphur compound of the formula

$R^3 - S(=O)_n - R^4$

in which

$R^3$ and $R^4$ independently of one another denote straight − chain or branched $C_1-C_{12}$ − alkyl, hydroxy − $C_2-C_{12}$ − alkyl, or carboxy − $C_1-C_{12}$ − alkyl or phenyl, and furthermore

$R^3$ and $R^4$ together may represent $-CH=CH-CH=CH-$, $-(CH_2)_4-$, $-(CH_2)_5-$, $-(CH_2)_2-S-(CH_2)_2-$ or $-(CH_2)_2-O-(CH_2)_2-$,

$R^4$ may additionally denote hydrogen or $CO-C_1-C_{12}$ − alkyl and

n assumes the value 0 or 1.

**2.** Process according to Claim 1, characterized in that organic sulphur compounds of the formula

$R^{13} - S(=O)_n - R^{14}$

are employed in which

$R^{13}$ and $R^{14}$ independently of one another denote straight chain or branched $C_1-C_{12}$ − alkyl, hydroxy − $C_2-C_{12}$ − alkyl or carboxy − $C_1-C_{12}$ − alkyl, and where

$R^{14}$ additionally may denote $CO-C_1-C_6$ − alkyl and

n assumes the value 0 or 1.

**3.** Process according to Claim 2, characterized in that bis − (2 − hydroxyethyl) sulphide is employed.

**4.** Process according to Claim 1, characterized in that Raney nickel, Raney nickel-iron, Raney nickel-cobalt or Raney nickel-iron-cobalt is employed as catalyst.

**5.** Process according to Claim 1, characterized in that the reaction is carried out at a pH of 8-14.

**6.** Process according to Claim 1, characterized in that $\alpha,\beta$-unsaturated ketones of the formula

$R^5-CH=CH-CO-R^6$

are employed in which

$R^5$ and $R^6$ independently of one another denote straight-chain or branched $C_1-C_6$-alkyl, $C_2-C_6$-alkenyl, cyclopropyl, cyclopentyl, cyclohexyl, benzyl, phenylethyl or phenyl, where at least one of the radicals $R^5$ and $R^6$ represents phenyl and where furthermore at least one of the radicals $R^5$ and $R^6$ is monosubstituted to trisubstituted by halogen.

**7.** Process according to Claim 6, characterized in that $\alpha,\beta$-unsaturated ketones of the formula

$R^7 - CH=CH - CO - R^8$

are employed in which

$R^7$ denotes phenyl and

$R^8$ denotes straight-chain or branched $C_1-C_6$-alkyl, $C_2-C_6$-alkenyl, cyclopropyl, benzyl or phenyl,

where at least one of the radicals $R^7$ and $R^8$ is monosubstituted to trisubstituted by halogen.

**8.** Process according to Claim 6, characterized in that 5-halophenyl-2,2-dimethyl-pent-4-en-3-ones or 6-halophenyl-3,3-dimethyl-hex-5-en-4-ones are employed.

## Revendications

**1.** Procédé d'hydrogénation catalytique de cétones à non-saturation $\alpha,\beta$ de formule

$R^1-CH=CH-CO-R^2$,

dans laquelle

$R^1$ et $R^2$ représentent, indépendamment l'un de l'autre, un groupe alkyle en $C_1$ à $C_{12}$ ou hydroxyalkyle en $C_2$ à $C_{12}$ à chaîne droite ou ramifiée, un groupe alcényle en $C_2$ à $C_{12}$ à chaîne droite ou ramifiée, un groupe cycloalkyle en $C_3$ à $C_8$, cycloalcényle en $C_3$ à $C_8$, aralkyle en $C_7$ à $C_{14}$ ou aryle en $C_6$ à $C_{12}$, l'un au moins des restes $R_1$ et $R_2$ étant substitué une à trois fois par un halogène,

pour former les cétones saturées correspondantes de formule :

$R^{11}-CH_2-CH_2-CO-R^{12}$

dans laquelle

$R^{11}$ et $R^{12}$ ont respectivement la définition de $R^1$ et $R^2$, sous réserve que le groupe alcényle et le groupe cycloalcényle soient hydrogénés en le groupe alkyle et respectivement cycloalkyle correspondant,

caractérisé en ce qu'on utilise un catalyseur contenant du nickel et on opère en phase liquide à une pression de $H_2$ de 3 à 200 bars et en présence d'un composé organique de soufre de formule

$R^3 - S(=O)_n - R^4$

dans laquelle

$R^3$ et $R^4$ représentent indépendamment l'un de l'autre un groupe alkyle en $C_1$ à $C_{12}$, hydroxyalkyle en $C_2$ à $C_{12}$ ou carboxyalkyle en $C_1$ à $C_{12}$, à chaîne droite ou ramifiée, ou un groupe phényle, et en outre

$R^3$ et $R^4$ peuvent représenter ensemble un groupe $-CH=CH-CH=CH-$, $-(CH_2)_4-$, $-(CH_2)_5-$, $-(CH_2)_2-S-(CH_2)_2-$ ou $-(CH_2)_2-O-(CH_2)_2-$,

$R^4$ peut représenter en outre l'hydrogène ou un groupe CO-alkyle en $C_1$ à $C_{12}$, et

n prend la valeur 0 ou 1.

**2.** Procédé suivant la revendication 1, caractérisé en ce qu'on utilise des composés organiques du soufre de formule

$R^{13} - S(=O)_n - R^{14}$

dans laquelle

$R^{14}$ peut représenter en outre un groupe CO-alkyle en $C_1$ à $C_6$ et

n prend la valeur 0 ou 1.

**3.** Procédé suivant la revendication 2, caractérisé en ce qu'on utilise le sulfure de bis-(2-hydroxyéthyle).

**4.** Procédé suivant la revendication 1, caractérisé en ce qu'on utilise comme catalyseur le nickel de Raney, le fer-nickel de Raney ou le cobalt-fer-nickel de Raney.

**5.** Procédé suivant la revendication 1, caractérisé en ce qu'on opère à un pH de 8 à 14.

**6.** Procédé suivant la revendication 1, caractérisé en ce qu'on utilise des cétones à non-saturation $\alpha,\beta$ de formule

$R^5-CH=CH-CO-R^6$

dans laquelle

$R^5$ et $R^6$ représentent indépendamment l'un de l'autre un groupe alkyle en $C_1$ à $C_6$ à chaîne droite ou ramifiée, alcényle en $C_2$ à $C_6$, cyclopropyle, cyclopentyle, cyclohexyle, benzyle, phényléthyle ou phényle, l'un au moins des restes $R^5$ et $R^6$ représentant un reste phényle et en outre, l'un au moins des restes $R^5$ et $R^6$ étant substitué une à trois fois par un halogène.

**7.** Procédé suivant la revendication 6, caractérisé en ce qu'on utilise des cétones à non-saturation $\alpha,\beta$ de formule

$R^7 - CH=CH - CO - R^8$

dans laquelle

$R^7$ désigne un groupe phényle et

$R^8$ est un groupe alkyle en $C_1$ à $C_6$ à chaîne droite ou ramifiée, alcényle en $C_2$ à $C_6$, cyclopropyle, benzyle ou phényle,

l'un au moins des restes $R^7$ et $R^8$ étant substitué une à trois fois par un halogène.

**8.** Procédé suivant la revendication 6, caractérisé en ce qu'on utilise des 5-halogénophényl-2,2-diméthyl-pent-4-ène-3-ones ou des 6-halogénophényl-3,3-diméthyl-hex-5-ène-4-ones.